# EUROPÄISCHE PATENTANMELDUNG

(11) **EP 1 787 683 A2**
(43) Veröffentlichungstag der Anmeldung: **23.05.2007**
(21) Anmeldenummer: 06021109.1
(22) Anmeldetag: 07.10.2006
(51) Int. Cl.: A61Q 5/08, A61K 8/22, A61K 8/23, A61K 8/24, A61K 8/19, A61K 8/42, A61K 8/37, A61K 8/365

(54) **Wasserfreie Zusammensetzungen und daraus herstellbare Mittel**

(30) Priorität: 09.11.2005 DE 102005053850
(71) Anmelder: HENKEL KOMMANDITGESELLSCHAFT AUF AKTIEN, 40589 Düsseldorf (DE)
(72) Erfinder: Höffkes, Horst, 40595 Düsseldorf (DE); Seiler, Martina, 47228 Duisburg (DE); Brockmann, Claudia, 40627 Düsseldorf (DE); Pauli, Kristin, 42119 Wuppertal (DE)

(57) **Zusammenfassung**

Es werden stabile wasserfreie Zusammensetzungen beschrieben, die neben mindestens einer festen Peroxoverbindung und mindestens einem alkalisch reagierenden Salz zusätzlich eine Kombination aus
- mindestens einem nicht hydroxylierten Fettsäureester mit einem Schmelzpunkt von höchstens 50°C und
- mindestens einer C₁₀- bis C₃₀-Fettsäure, die mindestens eine zusätzliche Hydroxygruppe trägt bzw. Derivate davon.

Aus dieser wasserfreien Zusammensetzung lassen sich stabile Mittel zur Aufhellung keratinhaltiger Fasern herstellen, die eine hervorragende Aufhellung bei gleichzeitig besserem Formerhalt und ästhetischem Gesamterscheinungsbild der Fasern bewirken.

## Beschreibung

Die vorliegende Erfindung betrifft wasserfreie Zusammensetzungen, die neben mindestens einer festen Peroxoverbindung und mindestens einem alkalisch reagierenden Salz zusätzlich eine Kombination aus mindestens einem Fettsäureester mit einem Schmelzpunkt von höchstens 50°C und mindestens einer C₁₀- bis C₃₀-Fettsäure mit mindestens einer zusätzlichen Hydroxygruppe enthalten. Ebenso betrifft die Erfindung aus besagter wasserfreier Zusammensetzung herstellbare Mittel für keratinhaltige Fasern und die Verwendung dieser Mittel zum Bleichen keratinhaltiger Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Ferner werden häufig aufhellende Verfahren, die sogenannten Blondierverfahren, angewendet. Die in den Bleichmitteln enthaltenen Oxidationsmittel wirken auf den natürlichen Haarfarbstoff Melanin und gegebenenfalls auf in der Faser befindliche synthetische Farbstoffe oxidativ ein und verursachen dadurch eine Aufhellung der Haarfarbe. Die Grundlagen der Blondier- und oxidativen Färbeverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Aufhellen bzw. Blondieren menschlicher Haare - insbesondere für die Strähnchenapplikation - werden üblicherweise wasserfreie, feste oder pasten- bis ölförmige Zubereitungen enthaltend feste Oxidationsmittel und mindestens ein alkalisch reagierendes Salz unmittelbar vor der Anwendung mit einer wässrigen, verdünnten Wasserstoffperoxidlösung vermischt. Diese Mischung wird dann auf das Haar aufgebracht und nach einer bestimmten Einwirkzeit wieder ausgespült.

Bei Verwendung besagter wasserfreier Zubereitungen ergibt sich die Schwierigkeit, die festen Bestandteile derart in den pasten- bzw.- ölförmigen Träger einzuarbeiten, dass sich einerseits das Trägermaterial nicht in mehrere Phasen auftrennt und andererseits die festen Oxidationsmittel nicht sedimentieren. Die wasserfreien Zusammensetzungen sollten sich schnell mit einer wässrigen Zusammensetzung zu einer stabilen Emulsion mischen lassen.

Herkömmliche wasserfreie, pasten- oder ölförmige Träger basieren auf flüssigen Esterkomponenten, wie z.B. Octyl- bzw. Isopropylstearat. Zur Verdickung setzt man üblicherweise Silikagel oder Kohlenwasserstoffe wie Vaseline oder Paraffinwachse zu. Die herkömmlichen Träger sind jedoch in ihrer Stabilität verbesserungswürdig.

Bei Verwendung von Vaseline oder Paraffinwachsen als Verdickungsmittel erfährt die keratinhaltige Faser zusätzlich eine verstärkte Belastung durch Ablagerung, wirkt schwer, unästhetisch fettig und lässt sich dadurch erschwert frisieren.

Aufgabe der vorliegenden Erfindung ist es daher, die Stabilität von wasserfreien Zusammensetzungen mit festen Peroxoverbindungen zu verbessern und eine schnelle Mischung von wasserfreien Zusammensetzungen mit wässrigen Zusammensetzungen unter Erhalt einer stabilen Emulsion zu ermöglichen. Wenn keratinhaltige Fasern mit einem aus der wasserfreien Zusammensetzung hergestellten Aufhellmittel behandelt wurden, sollen diese Fasern danach neben einer hervorragenden Aufhellung ebenso verbesserte Eigenschaften bezüglich ästhetischer Wirkung, Formerhalt, bzw. Frisurenerhalt, besitzen.

Unter keratinhaltigen Fasern werden erfindungsgemäß Pelze, Wolle, Federn und insbesondere menschliche Haare verstanden.

Es wurde nun überraschenderweise gefunden, daß die nachfolgend definierte wasserfreie Zusammensetzung die Aufgabe in hervorragendem Maße löst.

Ein erster Gegenstand der vorliegenden Erfindung sind wasserfreie Zusammensetzungen, enthaltend mindestens eine feste Peroxoverbindung und mindestens ein Alkalisierungsmittel, dadurch gekennzeichnet, daß sie zusätzlich
(A1) mindestens einen nicht hydroxylierten Fettsäureester mit einem Schmelzpunkt von höchstens 50°C, insbesondere von höchstens 30°C, und
(A2) mindestens eine C₁₀- bis C₃₀-Fettsäure mit mindestens einer zusätzlichen
Hydroxygruppe und/oder ein Derivat davon enthält.

Die festen Peroxoverbindungen der erfindungsgemäßen wasserfreien Zusammensetzungen sind solche Peroxoverbindungen, die keine Anlagerungsprodukte von Wasserstoffperoxid an andere Komponenten darstellen. Die Auswahl der in den erfindungsgemäßen Mitteln potentiell enthaltenen Peroxoverbindungen unterliegt darüber hinaus prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Magnesium- und Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Peroxoverbindungen sind in den erfindungsgemäßen Zusammensetzungen bevorzugt in Mengen von 1 bis 40 Gew.-%, insbesondere in Mengen von 2 bis 30 Gew.-%, enthalten.

Die wasserfreien Zusammensetzungen enthalten mindestens ein Alkalisierungsmittel. Erfindungsgemäß können die dem Fachmann für Bleich- bzw. Aufhellmittel bekannten, üblichen Alkalisierungsmittel wie Ammonium-, Alkalimetall- und Erdalkalimetallhydroxide, -carbonate, -hydrogencarbonate, -hydroxycarbonate und -carbamide, sowie Alkaliphosphate und Alkalimetasilikate verwendet werden. Die erfindungsgemäßen wasserfreien Zusammensetzungen enthalten die Alkalisierungsmittel bevorzugt in Mengen von 0.2 bis 25 Gew.-%, insbesondere 0.5 bis 10 Gew.-%.

Ester von nicht hydroxylierten C₆- bis C₃₀-Alkylmonocarbonäuren mit C₂- bis C₃₀-Monoalkoholen eignen sich erfindungsgemäß bevorzugt als Fettsäureester (A1). Bevorzugt sind die Monoester der Fettsäuren mit Monoalkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Estern sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyloleat (Cetiol^{®}), Laurinsäurehexylester (Cetiol^{®} A), Myristylmyristat (Cetiol^{®} MM), Cetearylisononanoat (Cetiol^{®} SN), Ölsäuredecylester (Cetiol^{®} V).

Die erfindungsgemäßen wasserfreien Zusammensetzungen enthalten die nicht hydroxylierten Fettsäureester (A1) bevorzugt in einer Menge von 2 bis 60 Gew.-%, besonders bevorzugt von 5 bis 30 Gew.-%, jeweils bezogen auf das Gewicht der wasserfreien Zusammensetzung.

Die C₁₀- bis C₃₀-Fettsäure mit mindestens einer zusätzlichen Hydroxygruppe (A2) bzw. deren Derivate können sich von einer gesättigten oder ungesättigten Fettsäure ableiten die bevorzugt ein bis drei zusätzliche Hydroxygruppen tragen. Bevorzugt handelt es sich erfindungsgemäß um gesättigte Fettsäuren bzw. Derivate davon.

Wiederum bevorzugt eignen sich als Fettsäure gemäß (A2) die Monohydroxy-C₁₀- bis C₃₀-Carbonsäuren und deren Derivate. Als Derivate eignen sich insbesondere die Ester mit mindestens einem Vertreter der Fettalkohole, insbesondere derjenigen Fettalkohole, die unter den im Zusammenhang mit den Estern (A1) genannten Fettalkoholanteilen erwähnt werden.
Dabei wird die C₁₀- bis C₃₀-Fettsäure mit mindestens einer zusätzlichen Hydroxygruppe insbesondere ausgewählt aus Verbindungen der Formel (I) und/oder deren physiologisch verträglichen Salzen, 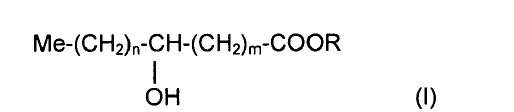 worin
R steht für ein Wasserstoffatom oder eine C₈- bis C₂₂-Alkylgruppe,
m eine ganze Zahl von 0 bis 27 und
n eine ganze Zahl von 0 bis 27 bedeuten, sowie
die Summe m + n eine ganze Zahl von 7 bis 27 beträgt.

Dabei ist es erneut bevorzugt, wenn die C₁₀- bis C₃₀-Fettsäure mit mindestens einer zusätzlichen Hydroxygruppe ausgewählt wird aus solchen Verbindungen der Formel (I) bzw. deren physiologisch verträglichen Salzen, die durch die Parameter R bedeutet ein Wasserstoffatom oder eine C₈- bis C₂₂-Alkylgruppe,
m eine ganze Zahl von 2 bis 27,
n eine ganze Zahl von 0 bis 27, sowie
die Summe m + n eine ganze Zahl von 7 bis 27
   definiert sind.

Solche C₁₀- bis C₃₀-Fettsäuren mit mindestens einer zusätzlichen Hydroxygruppe bzw. deren Derivate sind beispielsweise 12-Hydroxystearinsäure, 3-Hydroxypalmitinsäure, 12-Hydroxylaurinsäure, 16-Hydroxypalmitinsäure und 17-Hydroxypalmitinsäure bzw. deren physiologisch verträglichen Salze.

Ganz besonders bevorzugt ist die C₁₀- bis C₃₀-Fettsäure mit mindestens einer zusätzlichen Hydroxygruppe bzw. deren Derivate 12-Hydroxystearinsäure und/oder eines ihrer Derivate und/oder eines ihrer physiologisch verträglichen Salze. 12-Hydroxystearinsäurederivate sind als Esterderivat insbesondere in hydriertem Rizinusöl enthalten und als solches beispielsweise als Handelsprodukte Cutina^{®} HR (INCI-Bezeichnung: Hydrogenated Castor Oil; Fa. Cognis), Rilanit^{®} HT und Rilanit^{®} GTH (Fa. Cognis) erhältlich.

Die erfindungsgemäßen wasserfreien Zusammensetzungen enthalten die C₁₀- bis C₃₀-Fettsäuren mit mindestens einer zusätzlichen Hydroxygruppe bzw. deren Derivate bevorzugt in einer Menge von 0.1 bis 5 Gew.-%, besonders bevorzugt von 0.5 bis 3 Gew.-%, jeweils bezogen auf das Gewicht der wasserfreien Zusammensetzung.

Die erfindungsgemäßen wasserfreien Zusammensetzungen enthalten bevorzugt im Gewichtsverhältnisbereich der nicht hydroxylierten Fettsäureestern (A1) zu den C₁₀- bis C₃₀-Fettsäuren mit mindestens einer zusätzlichen Hydroxygruppe bzw. deren Derivaten (A2) von 10 zu 1 bis 60 zu 1.

Weiter verbessert werden die wasserfreien Zusammensetzungen, durch einen Zusatz mindestens eines Vertreters der Gruppe, bestehend aus Partialglyceriden, Fettalkoholen und Guerbetalkoholen, insbesondere mindestens eines Vertreters der der Gruppe bestehend aus Fettalkoholen und Guerbetalkoholen. Der besagte Zusatz ist bevorzugt in einer Menge von 0.1 bis 20 Gew.-%, besonders bevorzugt von 0.5 bis 10 Gew.-%, jeweils bezogen auf das Gewicht der wasserfreien Zusammensetzung, enthalten.

Partialglyceride, also Monoglyceride, Diglyceride und deren technische Gemische können herstellungs-bedingt noch geringe Mengen Triglyceride enthalten. Die Partialglyceride folgen vorzugsweise der Formel (II), 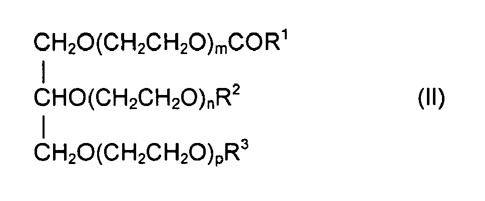 in der COR¹ für einen linearen oder verzweigten, gesättigten und/oder ungesättigten Acylrest mit 6 bis 22, vorzugsweise 12 bis 18 Kohlenstoffatomen, R² und R³ unabhängig voneinander für COR¹ oder OH und die Summe (m+n+p) für 0 oder Zahlen von 1 bis 100, vorzugsweise 5 bis 25 steht, mit der Maßgabe, daß mindestens einer der beiden Reste R² und R³ OH bedeutet.

Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen.

Bevorzugte Fettalkohole sind primäre aliphatische Alkohole der Formel (III),

R¹OH (III)

in der R¹ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind Fettalkoholmischungen, die Fettalkohole mit 12 bis 18 Kohlenstoffatomen enthalten, wie beispielsweise solche Mischungen, die aus Kokos-, Palm-, Palmkernöl oder Talgfett, insbesondere aus Kokosöl oder Talgfett, gewonnen werden.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20): Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Besonders bevorzugt besitzen die Fett- bzw. Guerbetalkohole der erfindungsgemäßen wässrigen Zusammensetzung einen Schmelzpunkt von höchstens 50°C, insbesondere höchstens 30°C.

Optional enthalten die erfindungsgemäßen wasserfreien Zusammensetzungen mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- anionische Alkyl- und/oder Alkenyl-Oligoglykosidcarboxylate, -sulfate, -phosphate und/oder -isethionate, die sich von Alkyl- und/oder Alkenyloligoglykosiden der allgemeinen Formel (IV) ableiten,

   R-O-(G)ₚ (IV)

   mit der Bedeutung
   R C₆₋₂₂-Alkyl oder C₆₋₂₂-Alkenyl,
   G Glykosideinheit, die sich von einem Zucker mit 5 oder 6 Kohlenstoffatomen ableitet,
   p Zahl von 1 bis 10.
   Bevorzugt sind Carboxylate, wie sie beispielsweise unter dem Handelsnamen Plantapon^{®} LGC (Alkylpolyglucosid-Carboxylat Natriumsalz; 30 % Aktivsubstanz; Fa. Cognis) vermarktet werden
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate, anionische Alkyl- und/oder Alkenyl-Oligoglykosidcarboxylate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.
   Beispiele für die in den erfindungsgemäßen Aufhellmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die erfindungsgemäßen wasserfreien Zusammensetzungen können zusätzlich mindestens einen weiteren Bleichverstärker, der von den festen Peroxoverbindungen verschieden ist, enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Aufhellleistung können der erfindungsgemäßen wasserfreien Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW und Portil^{®} W und von der Firma Akzo unter der Bezeichnung Britesil^{®} C20 vertrieben.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali-(insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (IV), 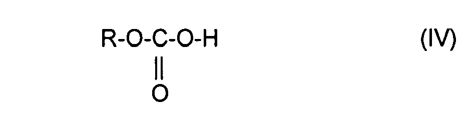 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (IV) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (IV) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (V) zu verwenden, 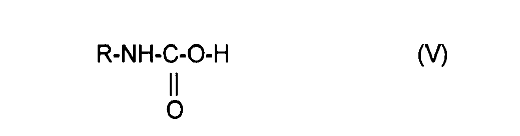 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (V) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (V) sind dadurch gekennzeichnet, daß der Rest R in Formel (V) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (VI) eingesetzt, 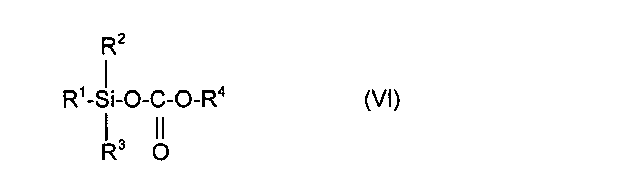 in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (VI) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße wasserfreie Zusammensetzungen dadurch gekennzeichnet sind, daß die Reste R¹, R² und R³ in Formel (VI) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ in der oben genannten Formel (VI) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (VII) in der erfindungsgemäßen wasserfreien Zusammensetzung enthalten sein, 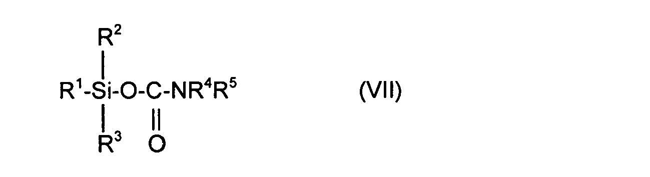 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (VII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (VII) ausgewählt sind aus Methyl-, Ethyl-, n-Propyl-, iso- Propyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (VII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Ein zweiter Gegenstand der Erfindung sind Mittel zur Aufhellung keratinhaltiger Fasern, insbesondere menschlicher Haare, welches vor der Anwendung aus mindestens einer wasserfreien Zusammensetzung gemäß erstem Erfindungsgegenstand und einer Zusammensetzung, enthaltend mindestens ein Oxidationsmittel gemischt wird.

Diese erfindungsgemäßen Aufhellmittel enthalten als wichtige Komponente mindestens ein Oxidationsmittel. Als erfindungsgemäßes Oxidationsmittel wird bevorzugt Wasserstoffperoxid verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon-n H₂O₂ (n ist eine positive ganze Zahl größer 0), Harnstoffperoxid und Melaminperoxid, mit der erfindungsgemäßen wasserfreien Zusammensetzung der ersten Erfindungsgegenstandes vermischt.

Die Konzentration einer Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von wasserfreier Zusammensetzung und Oxidationsmittelhaltiger Zusammensetzung liegen dabei üblicherweise im Bereich 1.5 zu 1 bis 1 zu 2.

Der pH-Wert der erfindungsgemäßen Aufhellmittel liegt bevorzugt in einem pH-Bereich von pH 2.5 bis 12.0, besonders bevorzugt von pH 7 bis 10.0.

In einer weiteren Ausführungsform enthalten die erfindungsgemäßen Aufhellmittel zusätzlich weitere strukturverbessernde Wirkstoffe. Diese Wirkstoffe sind bevorzugt in der wasserfreien Zusammensetzung des ersten Erfindungsgegenstandes enthalten, können aber ebenso in der Oxidationsmittelhaltigen Zusammensetzung enthalten sein.

Solche die Haarstruktur verbessernden Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbesserndes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropandiol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Aufhellmittel geeignet sind die Extrakte aus Mandel, Aloe Vera, Kokosnuß, Mango, Aprikose, Limone, Weizen, Kiwi, Melone und grünem Tee.

Wenn die Pflanzenextrakte in die wasserfreie Zusammensetzung des ersten Erfindungsgegenstandes eingearbeitet werden, ist es sinnvoll, die wässrigen Lösemittel zu entfernen.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin^{®} WQ (INCI-Bezeichnung: Laurdimonium Hydroxypropyl Hydrolyzed Wheat Protein) und Crotein^{®} Q (INCI-Bezeichnung: Hydroxypropyltrimonium Hydrolyzed Collagen) vertrieben werden, erfindungsgemäß besonders bevorzugt. Wenn die Proteinhydrolysate in die wasserfreie Zusammensetzung des ersten Erfindungsgegenstandes eingearbeitet werden, ist es sinnvoll, die wässrigen Lösemittel zu entfernen.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol^{®} A15 (INCI-Bezeichnung: Polyquaternium-2), Onamer^{®} M (INCI-Bezeichnung: Polyquaternium-1) und Merquat^{®} 100 (INCI-Bezeichnung: Polyquaternium-6), Polymer JR 400 (INCI-Bezeichnung: Polyquaternium-10) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen, Hexosen und Heptosen, wie die entsprechenden On- und Uronsäuren (Zuckersäuren), Zuckeralkohole, Zuckeramine, wie beispielsweise N-Glucosamin, und Glykoside, sowie mit C₄-C₃₀-Fettalkoholen veretherte Pentosen, Hexosen und Heptosen, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γ-lacton, Lactobionsäure, die Glucuronsäure und ihre Mono- beziehungsweise Dilactone, die Pangaminsäure, die Zuckersäure, die Mannozuckersäure und ihre Monobeziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere von Komponente (A2) verschiedene α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure und Glycerinsäure sind besonders bevorzugt. Weiterhin verbessern spezielle Phosphonsäuren und ihre Salze die Struktur keratinhaltiger Fasern. Erfindungsgemäß bevorzugte Phosphonsäuren sind die n-Octylphosphonsäure und die n-Decylphosphonsäure.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3a*S*,4*S*, 6a*R*)-2-Oxohexa-hydrothienol[3,4-d]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di- und Oligosacchariden, Serin, Milchsäure, Glycerinsäure, Niacinamid, Vitamin B6, Polyvinylpyrrolidon, Glucose, Gluconsäure und Biotin.

Die erfindungsgemäßen Mittel enthalten die strukturverbessernden Wirkstoffe bevorzugt in Mengen von 0,1 bis 5 Gew.-%, besonders bevorzugt in Mengen von 0,2 bis 2 Gew.-%.

Die erfindungsgemäßen Mittel können weiterhin alle für solche Zubereitungen bekannten Wirk-, Zusatz- und Hilfsstoffe enthalten.

Weiterhin können die erfindungsgemäßen Aufhellmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl, sowie Tocopherolacetat.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle,
- Cyclodextrine,
- Lösungsmittel und -vermittler wie Ethanol, Isopropanol, Ethylenglykol, Propylenglykol, Glycerin, Dimethylisosorbid und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein vierter Gegenstand der vorliegenden Erfindung ist ein Verfahren zur Aufhellung keratinhaltiger Fasern, bei dem die Fasern mit dem oben beschriebenen Mittel des zweiten Erfindungsgegenstandes behandelt werden.

Die folgenden Beispiele sollen den Gegenstand der vorliegenden Erfindung erläutern ohne ihn jedoch zu beschränken.

### Beispiele

### 1. Emulsionsbildung

Die Rezepturen 1 bis 4 gemäß Tabelle 1 wurden durch Vermischen der Rezepturbestendteile bei ca. 80° C nach bekanntem Verfahren hergestellt.

**Tabelle 1: Wasserfreie Zusammensetzungen**

| Rohstoff | Rezepturnr. / Menge in g | | | |
|---|---|---|---|---|
| | 1 | 2 | 3 | 4 |
| (NH₄)₂S₂O₈ | 12,5 | 12,5 | 12,5 | 12,5 |
| Britesil^{®} C 20 | 3,5 | 3,5 | 3,5 | 3,5 |
| Cutina^{®} HR | 1,0 | 1,0 | 1,0 | 1,0 |
| Elfan^{®} AT 84 | 2,0 | - | - | - |
| Texapon^{®} K 12 | - | 2,0 | - | - |
| Cetyltrimethyl-ammoniumbromid | - | - | 2,0 | 2,0 |
| Cetiol^{®} 868 | 10,0 | 10,0 | 10,0 | 10,0 |
| Lorol^{®} technisch | 5,0 | 5,0 | 5,0 | - |
| Cutina^{®} GMS | - | - | - | 5,0 |

Pro Mischung wurde 1 Gewichtsteil einer Rezeptur aus Tabelle 1 mit 9 Gewichtsteilen Wasser bei 80°C gemischt. Die Emulsionsstabilität wurde unmittelbar nach der Herstellung und nach 1 Tag beurteilt:

**Tabelle 2: Ergebnisse des Stabilitätstests der resultierenden Emulsion**

| **Rez. - Nr.** | **Stabilität nach Herstellung** | **nach 1 Tag** |
|---|---|---|
| 1 | stabil, homogen | stabil, homogen |
| 2 | stabil, homogen | stabil, homogen |
| 3 | stabil, homogen | stabil, homogen |
| 4 | stabil, homogen | stabil, homogen |

### 2. Anwendung der erfindungsgemäßen wasserfreien Zusammensetzungen

| **Rohstoff** | **Rezeptur-Nr. / g Einwaage** | | |
|---|---|---|---|
| | **1** | **2** | **3** |
| Ammoniumperoxidisulfat | 12,5 | - | 2,0 |
| Kaliumperoxidisulfat | - | 4,5 | 2,0 |
| Natriumperoxidisulfat | - | 5,5 | 6,0 |
| Britesil^{®} C20 ¹ | - | 4,5 | 3,5 |
| Texapon^{®} K12P ² | - | 1,0 | - |
| Elfan^{®} AT 84 ³ | 1,0 | - | - |
| Cetyltrimethylammoniumbromid | - | - | 1,0 |
| Cetiol^{®} 868 ⁴ | 8,1 | 5,0 | 5,0 |
| Titandioxid Kronos^{®} 1171 ⁵ | 0,3 | 0,3 | 0,3 |
| Cutina^{®} HR ⁶ | 0,2 | 0,2 | 0,2 |
| Lorol^{®} technisch ⁷ | - | 2,0 | - |
| Cutina^{®} GMS ⁸ | - | - | 0,4 |
| Eutanol^{®} G ⁹ | - | 0,4 | - |
| EDTA, Di-Natriumsalz | 0,5 | 0,5 | 0,5 |
| Aerosil^{®} 200 ¹⁰ | 0,1 | 0,1 | 0,1 |
| Cekol^{®} 50.000 ¹¹ | 1,0 | 1,0 | 1,0 |
| Trinatriumphosphat | 1,3 | - | - |

| | | | |
|---|---|---|---|
| ¹ Wasserglas (INCI-Bezeichnung: Sodium Silicate) (PQ Europe) ² Dodecylsulfat, Natriumsalz (pulverförmig, INCI-Bezeichnung: Sodium Lauryl Sulfate) (Cognis) ³ Kokosfettsäureisethionat, Natriumsalz (pulverförmig, INCI-Bezeichnung: Sodium Cocoyl Isethionate) (Akzo Nobel) ⁴ Stearinsäure-2-ethylhexylester (INCI-Bezeichnung: Ethylhexyl Stearate) (Cognis) ⁵ Anataspigment (INCI-Bezeichnung: Cl 77891 Titanium Dioxide) (Kronos) ⁶ gehärtetes Rizinusöl (fest, INCI-Bezeichnung: Hydrogenated Castor Oil) (Cognis) ⁷ Mischung aus C₁₂- bis C₁₈ Fettalkoholen (INCI-Bezeichnung: Coconut Alcohol) (Cognis) ⁸ Glycerinmonostearat (fest, INCI-Bezeichnung: Glyceryl Stearate) (Cognis) ⁹ 2-Octyldodecanol (INCI-Bezeichnung: Octyldodecanol) (Cognis) ¹⁰ Kieselsäure pyrogen, hydrophil (INCI-Bezeichnung: Silica) (Degussa) ¹¹ Carboxymethylcellulose, Natrium Salz (pulverförmig, INCI-Bezeichnung: Cellulose Gum) (CP Kelco) | | | |

Je eine aus den Rezepturen 1 bis 3 wurden im Gewichtsverhältnis 1:3 mit der Oxidationsmittelhaltigen Zusammensetzung gemäß Tabelle 3 unter Erhalt eines anwendungsfertigen Bleichmittels gemischt.

**Tabelle 3: Oxidationsmittelhaltige Zusammensetzung**

| | |
|---|---|
| Dipicolinsäure | 0.10 Gew.% |
| Na₂H₂P₂O₇ | 0.03 Gew.% |
| Turpinal^{®} SL¹² | 1.50 Gew.% |
| Texapon^{®}N 28 ¹³ | 2.00 Gew.% |
| Dow Corning DB 110¹⁴ | 0.07 Gew.% |
| Aculyn^{®} 33¹⁵ | 12.00 Gew.% |
| Wasserstoffperoxid | 12.00 Gew.% |
| Ammoniak (25 %ige wäßrige Lösung) | 0.65 Gew.% |
| Wasser | ad 100 |

| | |
|---|---|
| ¹² 1-Hydroxyethan-1,1-diphosphonsäure (ca. 60% Aktivsubstanz; INCI-Bezeichnung: Etidronic Acid) (Cognis) ¹³ Natriumlaurylethersulfat (ca. 28% Aktivsubstanz; INCI-Bezeichnung: Sodium Laureth Sulfate) (Cognis) ¹⁴ nichtionogene Siliconemulsion (ca. 10% Aktivsubstanz; INCI-Bezeichnung: Dimethicone) (Dow Corning) ¹⁵ wässrige Acrylatdispersion (ca. 28% Aktivsubstanz; INCI-Bezeichnung: Acrylates Copolymer) (Rohm & Haas) | |

Die pH-Werte der Mischungen (Bleichmittel) waren wie folgt:

| Mischung mit Rezeptur-Nr. | pH-Wert |
|---|---|
| 1 | 7,5 |
| 2 | 9,6 |
| 3 | 9,4 |

Haarsträhnen (Fischbach & Miller 6923) wurden mit den Mischungen behandelt. Pro 1 g Haarsträhne wurden 4 g eines der hergestellten Bleichmittel auf die Haarsträhne appliziert und dort für 45 Minuten bei einer Temperatur von 32°C belassen. Danach wurde das Mittel mit Wasser von der Strähne gespült. Das Haar wurde abschließend getrocknet.

Für die Applikationsdauer eines Haarbehandlungsmittels von 30-45 Minuten sowie für den Ausspülvorgang wurde eine hinreichend stabile, gut ausspülbare Emulsion gebildet, die zu keiner erhöhten Beschwerung des Haares führt. Im Falle der Verwendung von Cetyltrimethylammoniumbromid wird zusätzlich eine Pflegewirkung erzielt.

Rezeptur 1 ergab eine mittelstarke Aufhellung, die der Aufhellung mit üblichen Marktprodukten ohne Oxidationsverstärker (Poly Blond^{®} Medium; Fa. Schwarzkopf-Henkel) vergleichbar war. Rezepturen 2 + 3 ergaben vergleichbare bis leicht bessere Aufhellungen als bei üblichen Marktprodukten mit Oxidationsverstärkern (Poly Blond^{®} Ultra; Fa. Schwarzkopf-Henkel).

## Patentansprüche

1. Wasserfreie Zusammensetzung, enthaltend mindestens eine feste Peroxoverbindung und mindestens ein Alkalisierungsmittel, **dadurch gekennzeichnet, daß** sie zusätzlich (A1) mindestens einen nicht hydroxylierten Fettsäureester mit einem Schmelzpunkt
von höchstens 50°C, insbesondere von höchstens 30°C, und (A2) mindestens eine C₁₀- bis C₃₀-Fettsäure mit mindestens einer zusätzlichen
Hydroxygruppe und/oder ein Derivat davon enthält.

2. Wasserfreie Zusammensetzung nach Anspruch 1, **dadurch gekennzeichnet, daß** die feste Peroxoverbindung ausgewählt wird aus mindestens einem Vertreter der Gruppe Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Magnesiumperoxid und Bariumperoxid.

3. Wasserfreie Zusammensetzung nach einem der Ansprüche 1 oder 2, **dadurch gekennzeichnet, daß** das Alkalisierungsmittel ausgewählt wird aus mindestens einem Vertreter der Gruppe Ammoniumcarbonat, Alkalimetallcarbonaten, Erdalkalimetallcarbonaten, Ammoniumhydrogencarbonat, Alkalimetallhydrogencarbonaten, Erdalkalimetallhadrogencarbonaten, Ammoniumcarbamid, Alkalimetallcarbamiden und Erdalkalimetallcarbamiden.

4. Wasserfreie Zusammensetzung nach einem der Ansprüche 1 bis 3, **dadurch gekennzeichnet, daß** der nicht hydroxylierte Fettsäureester (A1) ausgewählt wird aus Estern von nicht hydroxylierten C₆- bis C₃₀-Alkylmonocarbonäuren mit C₂- bis C₃₀-Monoalkoholen.

5. Wasserfreie Zusammensetzungen nach einem der Ansprüche 1 bis 4, **dadurch gekennzeichnet, daß** der Fettsäureester (A1) ausgewählt wird aus mindestens einem Vertreter der Gruppe, bestehend aus Isopropylmyristat, Isononansäure-C16-18-alkylester, 2-Ethylhexylpalmitat, Stearinsäure-2-ethylhexylester, Cetyloleat, Kokosfettalkohol-caprinat/-caprylat, n-Butylstearat, Oleylerucat, Isopropylpalmitat, Oleyloleat, Laurinsäurehexylester, Myristylmyristat, Cetearylisononanoate und Ölsäuredecylester.

6. Wasserfreie Zusammensetzung nach einem der Ansprüche 1 bis 5, **dadurch gekennzeichnet, daß** die C₁₀- bis C₃₀-Fettsäure mit mindestens einer zusätzlichen Hydroxygruppe aus Verbindungen der Formel (I) und/oder deren physiologisch verträglichen Salzen ausgewählt wird, 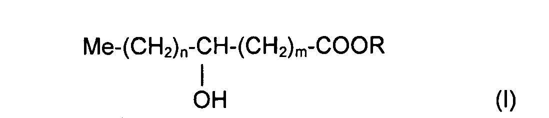 worin
R steht für ein Wasserstoffatom oder eine lineare C₈- bis C₂₂-Alkylgruppe,
m eine ganze Zahl von 0 bis 27 und
n eine ganze Zahl von 0 bis 27 bedeuten, sowie
die Summe m + n eine ganze Zahl von 7 bis 27 beträgt.

7. Wasserfreie Zusammensetzungen nach Anspruch 6, **dadurch gekennzeichnet, daß** in Formel (I)
R steht für ein Wasserstoffatom oder eine C₈- bis C₂₂-Alkylgruppe,
m eine ganze Zahl von 2 bis 27 und
n eine ganze Zahl von 0 bis 27 bedeuten, sowie
die Summe m + n eine ganze Zahl von 7 bis 27 beträgt.

8. Wasserfreie Zusammensetzung nach einem der Ansprüche 1 bis 7, **dadurch gekennzeichnet, daß** die C₁₀- bis C₃₀-Fettsäure mit mindestens einer zusätzlichen Hydroxygruppe bzw. deren Derivate 12-Hydroxystearinsäure und/oder eines ihrer Derivate und/oder eines ihrer physiologisch verträglichen Salze ist.

9. Wasserfreie Zusammensetzung nach einem der Ansprüche 1 bis 8, **dadurch gekennzeichnet, daß** sie zusätzlich mindestens einen Vertreter der Gruppe, bestehend aus Partialglyceriden, Fettalkoholen und Guerbetalkoholen, insbesondere mindestens einen Vertreter der der Gruppe bestehend aus Fettalkoholen und Guerbetalkoholen, enthält.

10. Wasserfreie Zusammensetzung nach Anspruch 9, **dadurch gekennzeichnet, daß** die Fettalkohole aus Fettalkoholen mit einem Schmelzpunkt von höchstens 50°C, insbesondere höchstens 30°C, ausgewählt werden.

11. Wasserfreie Zusammensetzung nach einem der Ansprüche 1 bis 10, **dadurch gekennzeichnet, daß** sie zusätzlich mindestens eine gegebenenfalls hydratisierte SiO₂-Verbindung enthält.

12. Wasserfreie Zusammensetzung nach Anspruch 11, **dadurch gekennzeichnet, daß** die gegebenenfalls hydratisierte SiO₂-Verbindung ein Silikat der Formel (SiO₂)ₙ(Na₂O)ₘ(K₂O)ₚ ist, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

13. Wasserfreie Zusammensetzung nach einem der Ansprüche 11 oder 12, **dadurch gekennzeichnet, daß** sie die gegebenenfalls hydratisierten SiO₂-Verbindungen in einer Menge von 0.05 bis 15 Gew.% enthält.

14. Wasserfreie Zusammensetzung nach einem der Ansprüche 1 bis 13, **dadurch gekennzeichnet, daß** zusätzlich mindestens ein Tensid enthalten ist.

15. Mittel zur Aufhellung keratinhaltiger Fasern, insbesondere menschlicher Haare, welches vor der Anwendung aus mindestens einer wasserfreien Zusammensetzung gemäß einem der Ansprüche 1 bis 14 und einer Zusammensetzung, enthaltend mindestens ein Oxidationsmittel gemischt wird.

16. Verfahren zur Aufhellung keratinhaltiger Fasern, **dadurch gekennzeichnet, daß** die Fasern mit einem Mittel nach Anspruch 15 behandelt werden.
